# EUROPEAN PATENT APPLICATION

(11) **EP 2 712 498 A1**
(43) Date of publication of application: **02.04.2014**
(21) Application number: 13185775.7
(22) Date of filing: 24.09.2013
(51) Int. Cl.: A01G 7/02, A01G 9/18, B01J 7/02, C07C 309/30

(54) **Apparatus for neutralisation of linear alkybenzene sulphonic acid**

(30) Priority: 27.09.2012 EP 12186358
(71) Applicant: The Procter & Gamble Company, Cincinnati, OH 45202 (US)
(72) Inventor: Brooker, John, Mark, Roger, Newcastle upon Tyne, Tyne and Wear NE3 5LP (GB); Somervilleroberts, Nigel, Patrick, Newcastle upon Tyne, Tyne and Wear NE20 9UJ (GB); Reid, Victor, Stuart, Newcastle upon Tyne, Tyne and Wear NE7 7LL (GB); Parmley, David, James, Gateshead, NE9 5BH (GB)
(74) Representative: Howard, Phillip Jan

(57) **Abstract**

Apparatus for making linear alkylbenzene sulphonate and method of making thereof.

## Description

### FIELD OF THE INVENTION

Apparatus for making linear alkylbenzene sulphonate and method of making thereof.

### BACKGROUND OF THE INVENTION

During granular detergent composition production, linear alkylbenzene sulphonic acid (HLAS) can be neutralised with carbonate. A by-product of this reaction is CO₂. This CO₂ is currently simply released into the atmosphere, which is wasteful and inefficient.

This inefficiency is least acceptable in developing markets. For every one part of raw material added, a certain amount is lost as CO₂ and not converted into useful neutralised LAS. Resources spent on raw materials, which are then lost as unused by-product from the reaction, cannot be spent on other necessities such as food or water. The key to maximizing the efficiency of any process is to (i) minimize any waste streams and to use those waste streams as raw materials for other processes and (ii) integrate as many processes together to maximize efficiency and re-use equipment for as many different processes as possible. This "verbund" approach can be as applicable to small-scale localized production of detergent products and other materials as to large-scale industrial production.

The use of CO₂ in greenhouses to encourage plant production is known and practiced commercially. Large greenhouses may have CO₂ addition systems to maintain an enhanced level of CO₂ in the growing area. Typically these use liquid CO₂ tanks and trickle feeders to maintain a suitable enhanced level of CO₂. These systems are complex and expensive and unsuitable for use in smaller installations in less-developed regions where liquid CO₂ is not readily available. Alternatively CO₂ can be generated by combustion. However, this is often not suitable as a means for boosting the CO₂ levels in growing spaces due to potential soot and smoke damage to the plants. Chemical reactions, e.g. acid/carbonate reactions, are efficient at producing pure CO₂ without soot or smoke and at high concentrations (which make it easier to collect the CO₂) but would need large amounts of reactants. Again this is not practical or economic if the only useful product from the reaction was CO₂.

Thus, there is a need in the art for means to improve the overall efficiency of detergent composition production. More specifically, there is a need to increase the overall efficiency of the HLAS neutralisation step by development of means to utilise waste products from this reaction productively.

The apparatus and process of the present invention overcome this problem by recycling the CO₂ produced to improve the growth of valuable plants, such as food crops. However, since the primary purpose of the chemical reactions is to make detergent products, then utilizing any the waste CO₂ is commercially and environmentally beneficial. An especially preferred approach is to combine detergent production with food and clean water production. This can be done, for example, in modified greenhouses with part of the greenhouse used for food crop growth and part of the same structure used for detergent and clean water production. Some of the raw materials used in detergent manufacture can be used to purify water.

### SUMMARY OF THE INVENTION

A first aspect of the present invention is an apparatus comprising;
a) a zone for reacting linear alkylbenzene sulphonic acid and carbonate to form linear alkyl benzene sulphonate and CO₂;
b) a CO₂ collection zone;
   wherein the CO₂ collection zone comprises at least one biological plant.

A second aspect of the present invention is a method of producing linear alkylbenzene sulphonate using the apparatus of the present invention comprising the steps of;
a) reacting linear alkylbenzene sulphonic acid and carbonate to form linear alkylbenzene sulphonate and CO₂;
b) collecting the CO₂ in a CO₂ collection zone;
wherein the CO₂ collection zone comprises at least one biological plant.

A third aspect of the present invention is the use of CO₂ formed from the reaction of linear alkylbenzene sulphonic acid with carbonate to increase the level of CO₂ in the presence of at least one plant so as to increase plant growth.

### BRIEF DESCRIPTION OF THE DRAWINGS

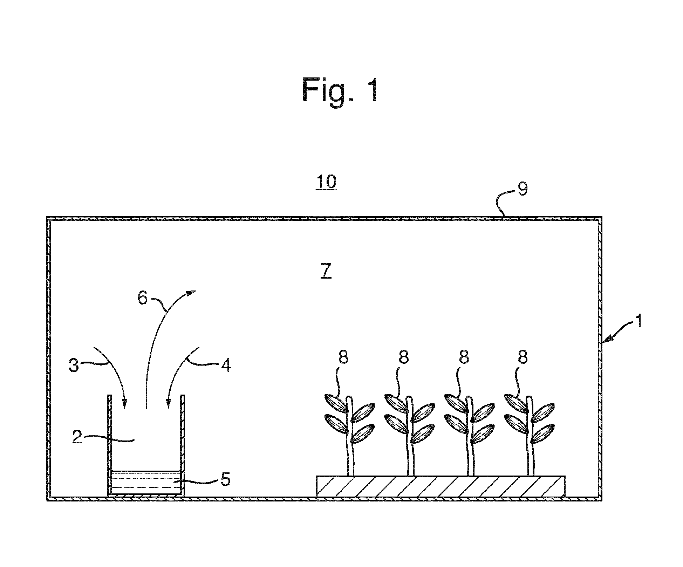

Fig. 1 discloses a preferred embodiment of the apparatus of the present invention.

### DETAILED DESCRIPTION OF THE INVENTION

### Apparatus

The present invention is to an apparatus (1) comprising;
a) a reaction zone (2) for reacting linear alkylbenzene sulphonic acid (3) and carbonate (4) to form linear alkybenzene sulphonate (5) and CO₂ (6);
b) a CO₂ collection zone (7);
wherein the CO₂ collection zone (7) comprises at least one biological plant (8).

The apparatus (1) of the present invention comprises a reaction zone (2) and a CO₂ collection zone (3).

The reaction zone (2) can comprise any means for holding linear alkybenzene sulphonic acid and carbonate whilst they react to form linear alkylbenzene sulphonate and CO₂ and then hold the alkylbenzene sulphonate product. The reaction zone (2) could comprise any suitable vessel or container to achieve this. The reaction zone (2) could comprise a reaction tank or even a batch reaction tank. The suitable vessel or container can be made of any suitable material for the addition of linear alkybenzene sulphoic acid and carbonate and the subsequent production of linear alkylbenzene sulphonate. The reaction zone (2) could comprise an open top vessel or container or can be closed. If it is closed then it must comprise a means for the release of CO₂ to the CO₂ collection zone (7). The reaction zone (2) must also comprise means for the addition of linear alkylbenzene sulphonic acid and carbonate and also means for the collection of linear alkybenzene sulphonate.

The apparatus (1) may also comprise a suitable linear alkylbenzene sulphonate storage area. The storage area may comprise a holding vessel such as a tank or comprise a flat bed or tray for example. The linear alkylbenzene sulphonate containing composition could be stored here whilst it dries following production.

The linear alkylbenzene sulphonic acid (3) could be any linear alkylbenzene sulphonic acid. Preferred linear alkybenzene sulphonic acid is described in more detail below.

The carbonate (4) can be any suitable carbonate. Preferred carbonates are discussed in more detail below.

The CO₂ collection zone (7) can comprise any suitable means to collect the CO₂. The CO₂ collection zone could be a storage tanker or container which is located away from the reaction zone (2). The CO₂ collection zone (7) could be a sealed unit (9) such that the CO₂ is collected within the collection zone and is isolated from the atmosphere external to the CO₂ collection zone (10). The reaction zone (2) could be present within the CO₂ collection zone (7), such that CO₂ produced from the reaction moves directly into the CO₂ collection zone. Alternatively, the reaction zone (2) and the CO₂ collection zone (7) are located away from each other, and the CO₂ is transported from the reaction zone (2) to the CO₂ collection zone (7) via a pipe or similar means.

The CO₂ collection zone (7) comprises at least one biological plant. By biological plant, we herein mean a multicellular living organism having roots and leaves, typically growing in a permanent site and that absorbs water and inorganic substances through the roots and synthesizes nutrients in the leaves by photosynthesis using chlorophyll. Preferred plants are described in more detail below.

Without wishing to be bound by theory, plants convert carbon dioxide in sugars (which they use as a food source) via a redox reaction called carbon fixation. Photosynthesis supplies the energy and electrons required for this reaction. The CO₂ produced by the reaction of linear alkylbenzene sulphonic acid and carbonate can be supplied to the plants in the CO₂ collection zone (7) and hence can be converted into a sugars by the plants and hence used as a food source by the plants. Increase in food source will increase the growth of the plants. These plants can then be used as a human food source or can be used for the manufacture of other products. Hence this limits the wastage of by-products from the reaction.

The CO₂ collection zone (7) may comprise between greater than 370ppm and up to and including 7000ppm CO₂, or even 750ppm to 6000ppm, or even 1000 to 6000ppm CO₂. Preferably the CO₂ collection zone (7) comprises between greater than 370ppm and up to and including 6000ppm CO₂ or even 750ppm to 6000ppm, or even 1000 to 6000ppm CO₂ during reaction of linear alkybenzene sulphonic acid with carbonate. The production of the CO₂ occurs during a short period of time, typically only 2-4 minutes in a common batch reaction. This means that the CO₂ needs to be retained in the collection zone for a period long enough to allow the plants to absorb the CO₂ from the atmosphere. Hence the rate of exchange of internal air within the collection zone (7) with the atmosphere external to the CO₂ collection zone (10) needs to be limited. This can be achieved for example wherein the CO₂ collection zone (7) is in the form of a sealed greenhouse where opportunities for exchange are reduced and controlled. However it preferred that the CO₂ collection zone (7) is not completely sealed as this can lead to excessively high humidities within the CO₂ collection zone that can cause harm to any plants therein. The rate of air exchange from the CO₂ collection zone (7) to outside of the CO₂ collection zone (10) may be less than 25% per hour, i.e. less than 25% of the air enclosed in the CO₂ collection zone (7) should exchange to the external environment (10) in one hour. The rate of air exchange from the CO₂ collection zone (7) to outside of the CO₂ collection zone (10) should be less than 20% per hour, or even less than 15% per hour or even less than 10% per hour. The rate of air exchange from the CO₂ collection zone (7) to outside of the CO₂ collection zone (10) should be at least 0.5% per hour, or even at least 1% per hour. Those skilled in the art will know how to determine the rate of air exchange.

### Linear alkylbenzene sulphonic acid

The linear alkybenzene sulphonic acid can be any linear alkylbenzene sulphonic acid, preferably C₁₀₋₁₃ alkyl benzene sulphonate. Suitable alkyl benzene sulphonate (LAS) is obtainable, by sulphonating commercially available linear alkyl benzene (LAB); suitable LAB includes low 2-phenyl LAB, such as those supplied by Sasol under the tradename Isochem® or those supplied by Petresa under the tradename Petrelab®, other suitable LAB include high 2-phenyl LAB, such as those supplied by Sasol under the tradename Hyblene®.

### Carbonate

Any carbonate can be used for the present invention. The carbonate could be sodium carbonate or magnesium carbonate. Alternatively sodium sesquicarbonate or sodium bicarbonate or any other carbonate source could be used.

### Biological plants

Any biological plants (8) can be present in the CO₂ collection zone (7). Preferably, the biological plant (8) is one edible for humans or comprises an edible part for humans or is used to make an edible substance for humans or a combination thereof. Preferably, the biological plant is a photoautotroph, i.e. a biological plant that is able to synthesise its own food source from CO₂ and water using energy from light (photosynthesis). Thus, in a preferred embodiment, the apparatus (1) comprises a light source. The light source could be a natural light source (i.e. light directly from the sun). The biological plant could comprise be a cereal crop or a fruit bearing plant, or a combination thereof. Suitable cereal crops could include, rice, maize, barley, wheat or a combination thereof. Suitable fruit bearing plants could comprise, citrus fruit plants, tomatoes, vines, such as grapes, berry plants or combinations thereof.

### Process

The present invention is also to a method of producing linear alkybenzene sulphonate using the apparatus of the present invention comprising the steps of;
a) reacting linear alkylbenzene sulphonic acid and carbonate to form linear alkylbenzene sulphonic acid and CO₂;
b) collecting the CO₂ in a CO₂ collection zone;
wherein the CO₂ collection zone comprises at least one biological plant.

Preferably, the rate of CO₂ production is between 0.25 and 250 kg/day, or even 0.35 and 200kg/day. The method could be a continuous process, or a batch process. Continuous processing is preferred where the reaction zone is outside of the CO₂ collection zone. Batch processing is preferred when the reaction zone (2) is inside the collection zone (7) itself. Batch processing is compatible, for example, with the concept of a small, local "verbund" operation making several different products.

Often, linear alkylbenzene sulphonate will contain a low level of volatile organic compounds which can be released along with the CO₂ during the neutralization. These volatile inorganics are undesired if the CO₂ is being used in a closed environment for increasing crop growth. However, it was surprisingly found that the concentration of volatile organic compounds can be reduced by simply laying the linear alkylbenzene sulphonate out in a thin layer, for example at most 5 cm in depth, or at most 2.5 cm in depth, in the presence of moderate heat, for example between 25 and 45°C over an extended period, for example at least one day, or between 1 and 5 days, or even between 2 and 3 days. This can be achieved by, for example, pouring the linear alkylbenzene sulphonate into a tray and leaving it in a greenhouse at a temperature of between 25 and 45°C for 2-3 days.

### Detergent composition

The linear alkylbenzene sulphonate can be used in a detergent composition, preferably a laundry detergent composition. The detergent composition can be a solid or liquid form. The detergent composition is preferably a solid granular laundry detergent composition. The laundry detergent composition can comprise other typical laundry detergent ingredients. Suitable detergent ingredients include: surfactants, including anionic, non-ionic and cationic surfactants, transition metal catalysts; imine bleach boosters; enzymes such as amylases, carbohydrases, cellulases, laccases, lipases, bleaching enzymes such as oxidases and peroxidases, proteases, pectate lyases and mannanases; source of peroxygen such as percarbonate salts and/or perborate salts, preferred is sodium percarbonate, the source of peroxygen is preferably at least partially coated, preferably completely coated, by a coating ingredient such as a carbonate salt, a sulphate salt, a silicate salt, borosilicate, or mixtures, including mixed salts, thereof; bleach activator such as tetraacetyl ethylene diamine, oxybenzene sulphonate bleach activators such as nonanoyl oxybenzene sulphonate, caprolactam bleach activators, imide bleach activators such as N-nonanoyl-N-methyl acetamide, preformed peracids such as N,N-pthaloylamino peroxycaproic acid, nonylamido peroxyadipic acid or dibenzoyl peroxide; suds suppressing systems such as silicone based suds suppressors; brighteners; hueing agents; photobleach; fabric-softening agents such as clay, silicone and/or quaternary ammonium compounds; flocculants such as polyethylene oxide; dye transfer inhibitors such as polyvinylpyrrolidone, poly 4-vinylpyridine N-oxide and/or co-polymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters and/or terephthalate polymers, polyethylene glycol including polyethylene glycol substituted with vinyl alcohol and/or vinyl acetate pendant groups; perfumes such as perfume microcapsules, polymer assisted perfume delivery systems including Schiff base perfume/polymer complexes, starch encapsulated perfume accords; soap rings; aesthetic particles including coloured noodles and/or needles; dyes; fillers such as sodium sulphate, although it may be preferred for the composition to be substantially free of fillers; carbonate salt including sodium carbonate and/or sodium bicarbonate; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose, and hydrophobically modified cellulose; carboxylic acid and/or salts thereof, including citric acid and/or sodium citrate; and any combination thereof.

The detergent composition can be used in a hand wash operation such as a laundry hand wash operation or in a machine wash operation such as a laundry machine wash operation.

### EXAMPLES

A greenhouse, made from polycarbonate panels and of dimensions 2.2m by 4m by 2m was prepared containing an array of 20 tomato plants distributed equally along both sides. All panel joints were sealed with tape to reduce the exchange rate of internal and external air. The measured carbon dioxide level was between 450 and 480 ppm. This high level is likely due to the urban setting of the test apparatus.

A mass of 2 kg of linear alkylbenzene sulphonic acid was mixed with 1kg of water in a batch tank. 3.5 kg of light sodium carbonate were then gradually stirred into the liquid mix with continuous stirring over a period of five minutes. Then 400g of Sokalan CP5 polyacrylate polymer solution and 2 kg of sodium sulphate were stirred into the mixture. This slurry was then spread out thinly on trays and left to dry in the greenhouse. This procedure was then repeated in a batch production manner.

During this time the measured carbon dioxide level increased from 474ppm to 2461ppm. This decreased gradually over time such that after 3 hours the level was down to 1342ppm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An apparatus (1) comprising;
a) a reaction zone (2) for reacting linear alkylbenzene sulphonic acid (3) and carbonate (4) to form linear alkylbenzene sulphonate (5) and CO₂(6);
b) a CO₂ collection zone (7);
wherein the CO₂ collection zone (7) comprises at least one biological plant (8).

2. The apparatus (1) according to claim 1, wherein the reaction zone (2) is present within the CO₂ collection zone (7).

3. The apparatus (1) according to any preceding claims, wherein the CO₂ collection zone (7) is a sealed unit (9) such that the CO₂ is collected within the collection zone and is isolated from the atmosphere external to the CO₂ collection zone (10).

4. The apparatus (1) according to any preceding claims, wherein the CO₂ collection zone (7) comprises between 370ppm and up to and including 6000ppm CO₂, or even 750ppm to 6000ppm, or even 1000 to 6000ppm CO₂.

5. The apparatus (1) according to any preceding claims, wherein the rate of CO₂ loss from the CO₂ collection zone (7) to outside of the CO₂ collection zone (10) is less than 25% per hour.

6. The apparatus (1) according to any preceding claims, wherein the biological plant (8) is one edible for humans or comprises an edible part for humans or is used to make an edible substance for humans or a combination thereof.

7. The apparatus (1) according to any preceding claims comprising a light source, preferably a natural light source.

8. The apparatus (1) according to any preceding claims comprising a linear alkylbenzene sulphonate storage area.

9. A method of producing linear alkylbenzene sulphonate according to any preceding claims comprising the steps of;
a) reacting linear alkylbenzene sulphonic acid and carbonate to form linear alkylbenzene sulphonate and CO₂;
b) collecting the CO₂ in a CO₂ collection zone;
wherein the CO₂ collection zone comprises at least one biological plant.

10. The method according to claim 9, wherein the rate of CO₂ production is between 0.25 and 250 kg/day, or even between 0.35 and 200kg/day.

11. The method according to any of claims 9-10, comprising the step of laying the linear alkylbenzene sulphonate out in a thin layer of less than 5 cm in depth at a temperature of between 25 and 35°C for at least one day.

12. The method according to any of claims 9-11, wherein the reaction of linear alkylbenzene sulphonic acid and carbonate is a continuous process, or a batch process.

13. The use of CO₂ formed from the reaction of linear alkylbenzene sulphonic acid with carbonate to increase the level of CO₂ in the presence of at least one plant so as to increase plant growth.
